# FASCICULE DE BREVET EUROPEEN

(11) **EP 0 777 537 B1**
(45) Date de publication et mention de la délivrance du brevet: **03.03.1999**
(21) Numéro de dépôt: 94917051.8
(22) Date de dépôt: 24.05.1994
(51) Int. Cl.: B09B 3/00, A61L 11/00

(54) **INSTALLATION POUR LE BROYAGE ET LA DECONTAMINATION DE DECHETS, NOTAMMENT HOSPITALIERS**
ANLAGE ZUR MAHLUNG UND DEKONTAMINIERUNG VON ABFÄLLEN, INSBESONDERE VON KRANKENHAUSABFÄLLEN .
FACILITY FOR GRINDING AND DECONTAMINATING WASTE, PARTICULARLY HOSPITAL WASTE

(43) Date de publication de la demande: 11.06.1997
(73) Titulaire: ETS LAJTOS S.A., 59100 Roubaix (FR)
(72) Inventeur: DESCAMPS, Pierre, F-59830 Wannehain (FR)
(74) Mandataire: Hénnion, Jean-Claude
(86) Numéro de dépôt international: FR9400611
(87) Numéro de publication internationale: WO9532063

(56) Documents cités:
- EP-A- 0 545 520
- WO-A-93/14795
- DE-C- 4 128 854
- DE-U- 9 113 557
- US-A- 4 050 899

## Description

La présente invention concerne une installation spécialement conçue pour réaliser le traitement de déchets et plus particulièrement de déchets présentant une certaine toxicité comme les déchets hospitaliers, en vue de leur décontamination. Elle concerne plus particulièrement une installation de ce type dans laquelle les déchets sont préalablement broyés, soumis à de la vapeur à haute température avant d'être évacués.

On connaît déjà, par les documents DE.4128854 et WO 93/14795 une installation de ce type qui comporte une enceinte supérieure d'alimentation des déchets, des moyens de broyage disposés dans la partie basse de cette enceinte et une enceinte inférieure de réception et d'évacuation des déchets broyés. Cette installation comporte également des moyens d'injection de vapeur au moins dans l'enceinte inférieure.

Dans ces documents l'évacuation des déchets broyés et décontaminés s'effectue grâce à un système de vis sans fin. Dans le document DE.4128854 ce système est disposé axialement dans le fond de l'enceinte inférieure et dans une canalisation prolongeant ledit fond. Cette canalisation qui comporte une fermeture étanche peut déboucher, en fin de cycle, sur une autre canalisation oblique qui est traversée par un système de vis sans fin .C'est cette vis sans fin qui permet le remplissage des chariots qui sont amenés à proximité de l'installation. Les déchets broyés et décontaminés sont ainsi évacués.

Il est à noter que l'enceinte inférieure comporte un mélangeur à pales qui est entraîné en rotation de sorte d'effectuer une agitation constante des déchets broyés pendant le cycle de traitement à la vapeur.

Selon le demandeur l'installation décrite dans ces documents présentent un certain nombre d'inconvénients du fait notamment de leur système d'évacuation des déchets. Dans le document DE.4128854 la présence du mélangeur et de la vis sans fin à l'intérieur de l'enceinte inférieure rend plus complexe la fabrication et en particulier augmente les problèmes d'étanchéité au niveau de la couronne dentée permettant la rotation du mélangeur. De plus la présence des deux canalisations d'évacuation augmente l'encombrement en hauteur de l'installation, sachant que le chariot d'évacuation doit être placé en-dessous de la seconde canalisation oblique.

Le but visé par le demandeur est de proposer une installation pour le broyage et la décontamination de déchets notamment hospitaliers qui pallie les inconvénients précités.

Ce but est parfaitement atteint par l'installation qui comporte de manière connue par le document DE.4128854 une enceinte supérieure d'alimentation des déchets, une enceinte inférieure de réception et d'évacuation des déchets broyés, des moyens de broyage intercalés entre les deux enceintes, des moyens d'injection de vapeur dans l'enceinte inférieure et des moyens de commande de l'ouverture et de la fermeture d'une trappe de déchargement du fond de l'enceinte inférieure.

De manière caractéristique, selon l'invention, l'installation comporte également un récipient de stockage des déchets broyés qui est perforé pour permettre le passage de l'eau et de la vapeur, qui est ouvert sur le dessus et qui est disposé dans l'enceinte inférieure sous les moyens de broyage; le fond dudit récipient de stockage des déchets comporte au moins une trappe de déchargement ; l'enceinte inférieure comporte un couvercle de fond ; et enfin l'installation comporte des moyens de commande de l'ouverture et de la fermeture de la ou des trappes de déchargement ainsi que des moyens de commande de l'ouverture et de la fermeture du couvercle de fond de l'enceinte inférieure.

Les déchets provenant des moyens de broyage tombent dans le récipient de stockage et s'accumulent dans celui-ci, la trappe de déchargement étant fermée. Lors du traitement thermique, la vapeur peut passer à travers les perforations du récipient de stockage et pénétrer dans l'ensemble des déchets contenus dans ledit récipient. Une fois le traitement de décontamination terminé, il suffit d'ouvrir le couvercle de fond, de placer le chariot sous le récipient de stockage et d'ouvrir la ou les trappes de déchargement pour libérer l'ensemble des déchets contenus dans le récipient qui tombent directement dans le chariot. Un récipient de stockage de ce type était connu par le document DE 91 13 557.5, mais ne comportait pas de trappe de déchargement.

De préférence le fond comporte deux trappes symétriques de déchargement, chacune étant pivotante par rapport à un axe de pivotement qui est monté selon l'un des bords du fond du récipient ; de plus les deux trappes de déchargement sont actionnées par un jeu de tringles commandé par un vérin disposé à l'extérieur de l'enceinte inférieure. Cette disposition particulière à deux trappes de déchargement symétrique permet une bonne répartition de la charge des déchets dans le récipient et surtout de diminuer encore la hauteur sous l'installation nécessaire pour le passage du chariot.

Dans le document antérieur DE.4128854, la configuration de l'enceinte inférieure telle qu'elle est représentée à la figure annexée devait nécessairement entraîner une certaine accumulation de déchets dans la partie basse et périphérique de l'enceinte inférieure, malgré la présence du mélangeur. Dans l'installation de l'invention, il n'y a pas transfert progressif des déchets vers une canalisation axiale comme dans le cas du document précité, l'ensemble des déchets accumulés dans le récipient de stockage tombant sous son propre poids directement dans le chariot après l'ouverture des trappes de déchargement.

Pour éviter tout risque d'accrochage des déchets sur les montants latéraux du récipient de stockage, lesdits montants sont de préférence inclinés de manière divergente depuis l'ouverture supérieure vers le fond du récipient.

Avantageusement les moyens d'injection de la vapeur dans l'enceinte inférieure comprennent un premier circuit autour du récipient de stockage des déchets et un second circuit qui est disposé dans le couvercle de fond.

Etant donné que les déchets broyés s'accumulent dans le récipient de stockage perforé, il peut se produire des écoulement de liquide depuis le récipient jusque dans le couvercle de fond. Ceci sera notamment le cas pour des déchets du type protection hygiénique ou couche culotte. Le second circuit disposé dans le couvercle de fond permet d'atteindre sans risque la décontamination totale de tous les déchets, y compris les liquides qui se sont écoulés du récipient de stockage.

Avantageusement les premier et second circuits précités sont connectés au même circuit d'alimentation en vapeur et sont raccordés l'un à l'autre lorsque le couvercle de fond est en position fermée.

Selon une variante de réalisation, le couvercle de fond est à basculement selon un axe horizontal et à verrouillage par rotation sur lui-même d'un angle déterminé ; dans ce cas le circuit d'alimentation disposé à l'intérieur de l'enceinte inférieure se termine par un premier embout de raccordement et le second circuit commence par un second embout de raccordement, les deux embouts étant conformés et disposés de telle sorte que après fermeture et rotation de verrouillage du couvercle l'un des embouts est emmanché dans l'autre de manière étanche.

Pour obtenir un bon fonctionnement de l'installation, il est nécessaire d'obtenir un équilibrage des pressions à l'intérieur des deux enceintes. Dans le document DE.4128854, ceci était obtenu en injectant de la vapeur dans chacun des trois éléments constitutifs de l'installation à savoir l'enceinte supérieure, l'enceinte inférieure et la zone sous les moyens de broyage.

De manière avantageuse , selon l'invention, l'installation comporte une cuve à l'intérieur de laquelle sont disposés, dans sa partie haute, l'enceinte supérieure et dans sa partie intermédiaire les moyens de broyage; l'enceinte inférieure est constituée par la partie basse de la cuve. Ainsi la vapeur qui est injectée dans l'enceinte inférieure, c'est-à-dire la partie basse de la cuve, se répand dans toute la cuve , sans qu'il soit nécessaire d'effectuer des alimentations distinctes depuis le haut jusqu'en bas de celle-ci.

Etant donné que le traitement de décontamination est un traitement thermique qui se situe aux environs de 138°C, ce traitement provoque une augmentation conséquente de la température de l'ensemble des éléments qui sont en contact avec la vapeur. Une fois qu'un lot déterminé de déchets a été broyé et décontaminé, il importe de refroidir la cuve jusqu'à une température suffisante pour que le couvercle de fond puisse être ouvert sans préjudice pour l'opérateur.

De préférence selon l'invention les moyens de refroidissement consistent en une rampe de distribution d'eau sous pression qui est disposée dans la partie haute de l'installation et qui distribue l'eau selon la face externe de l'enceinte supérieure en sorte que l'eau ruisselle jusque dans le couvercle de fond de l'enceinte inférieure sans pénétrer dans les moyens de broyage. Dans le document antérieur DE.4128854, le refroidissement était obtenu en créant le vide à l'intérieur de l'installation . Il a été trouvé par le demandeur qu'un refroidissement par eau du type précité est beaucoup plus simple à mettre en oeuvre et consomme beaucoup moins d'énergie et d'eau. L'eau qui est utilisée pour ce refroidissement est collectée dans le couvercle de fond et évacuée avec le liquide qui s'était éventuellement écoulé des déchets stockés. Selon une version préférée , l'enceinte supérieure et la cuve étant de forme cylindrique et coaxiales, la rampe de distribution d'eau est une rampe circulaire qui distribue l'eau dans l'espace annulaire compris entre l'enceinte supérieure et la cuve ; de plus les moyens de broyage sont d'une dimension inférieure au diamètre de la cuve ; ainsi l'eau ruisselle dans l'espace annulaire précité puis le long de la paroi interne de la cuve jusque dans le couvercle de fond, sans pénétrer dans les moyens de broyage.

A aucun moment l'eau de ruissellement n'est en contact avec les déchets.

Lors de la montée en température , il est nécessaire de contrôler la pression interne régnant dans les enceintes. Ceci oblige à évacuer vers l'extérieur une certaine quantité de vapeur en cas de surpression, afin d'atteindre la température recherchée. Etant donné que cette vapeur évacuée a été en contact avec les déchets, le circuit d'évacuation de cette vapeur comporte un filtre du type bactériologique , apte à éliminer toute contamination. Cependant même si la vapeur qui est finalement évacuée est exempte de décontamination, celle-ci se retrouve sur le média filtrant contenu dans le filtre.

Selon l'invention on remédie à cet inconvénient en ce que l'installation comporte un circuit de régénération du filtre comprenant une alimentation de vapeur et un récipient de stockage des condensats de régénération, ledit récipient de stockage débouchant dans l'enceinte inférieure ; de plus l'installation comporte un circuit de commandes qui est programmé en sorte d'injecter le contenu du récipient de stockage des condensats, uniquement après chargement des déchets dans l'enceinte supérieure. Ainsi on réalise à chaque cycle la régénération du média filtrant. Les condensats contaminés sont stockés dans le récipient de stockage et ne sont réinjectés dans l'enceinte inférieure que lors du traitement de broyage et décontamination du lot de déchets suivant. Ces condensats sont donc eux-mêmes décontaminés à l'occasion du traitement thermique de ce lot suivant.

La présente invention sera mieux comprise à la lecture de la description qui va être faite d'un exemple de réalisation d'une installation de broyage et de décontamination à double enveloppe et à déchargement gravitaire, illustré par le dessin annexé dans lequel :
La figure 1 est une représentation schématique de l'installation,
Les figures 2 et 3 sont des représentations schématiques du récipient de stockage respectivement en position fermée pour la figure 2 et en position ouverture pour la figure 3 e,
La figure 4 est une représentation schématique et partielle en coupe de l'installation et,
La figure 5 est une représentation schématique montrant le raccordement des deux circuits d'injection de vapeur.

L'installation qui va être décrite a pour fonction de traiter des déchets qui sont contaminés, notamment des déchets de provenance médicale ou chirurgicale collectés dans des hôpitaux. Un tel traitement consiste à broyer lesdits déchets puis à leur faire subir un traitement thermique par action de la vapeur saturée à une température de 138° pendant dix minutes.

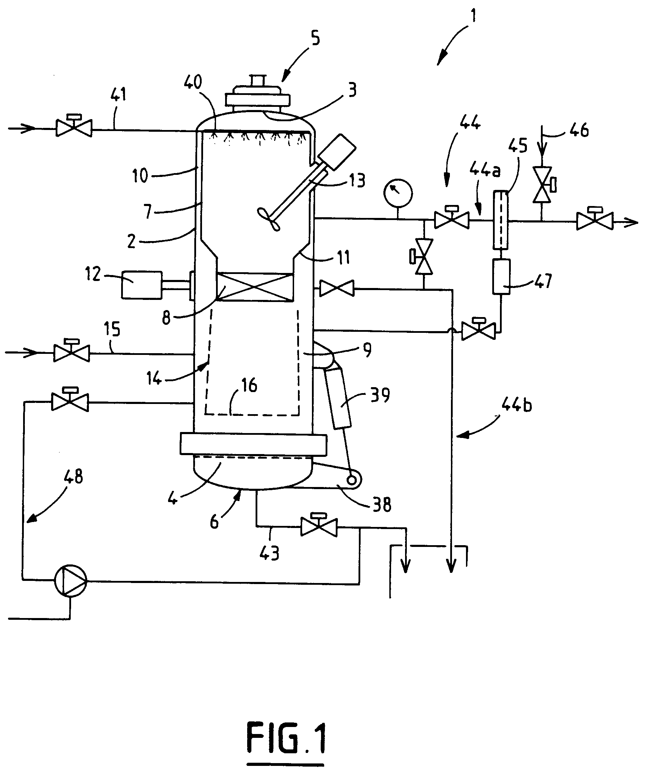

L'installation 1 représentée schématiquement à la figure 1 comporte une cuve cylindrique 2 d'axe vertical avec une ouverture de chargement 3 et une ouverture de déchargement 4 disposés respectivement à la partie haute et à la partie basse de la cuve 2. Chacune de ces deux ouvertures 3, 4 est fermée de manière étanche par un couvercle respectivement de chargement 5 et de fond 6.

A l'intérieur de la cuve 2 sont montés successivement, du haut en bas de la cuve 2, une enceinte supérieure 7 , des moyens de broyage 8 et une enceinte inférieure 9.

L'enceinte supérieure 7 est cylindrique, coaxiale avec la cuve 2. Cette enceinte supérieure 7 délimite avec la cuve 2 un espace annulaire 1O de faible dimension, pouvant faire de l'ordre d'un centimètre.

Cette enceinte supérieure 7 est ouverte vers le haut et vers le bas, débouchant dans sa partie basse sur les moyens de broyage 8 par une portion inclinée 11.

Les moyens de broyage 8 peuvent notamment consister dans des cylindres broyeurs d'axe horizontal disposés parallèlement l'un par rapport à l'autre et pourvus de dents de broyage aptes à déchiqueter les déchets qui sont chargés dans l'enceinte supérieure 7. Bien sûr ces cylindres tournent l'un par rapport à l'autre, et entraînent vers le bas de la cuve les déchets broyés.

L'enceinte inférieure 9 est constituée par la partie basse de la cuve 2, en-dessous des moyens de broyage. Dans cette enceinte inférieure 9, un récipient perforé 14 est fixé au-dessus du niveau du couvercle de fond 6.

La cuve 2 est alimentée en vapeur par la conduite 15 qui débouche dans la cuve 2 au niveau de l'enceinte inférieure 9. Plus précisément la conduite 15 se prolonge à l'intérieur de la cuve 2 sous la forme d'une conduite circulaire faisant le tour du récipient perforé 14 et également d'une conduite se prolongeant dans le couvercle de fond 6.

Le récipient perforé 14 est positionné de telle sorte que les déchets broyés par les moyens de broyage 8 tombent directement dans ledit récipient 14. Les perforations sont déterminées , en fonction des moyens de broyage 8, de manière à ce que tous les déchets broyés s'accumulent dans le récipient 14 sans passer par les perforations.

Le récipient perforé 14 comporte un fond escamotable. Plus précisément , dans l'exemple illustré aux figures , ce fond 16 est composé de deux trappes de déchargement 17a, 17b dont la fermeture et l'ouverture sont obtenues grâce à un jeu de tringles 18,19,2O commandé par un vérin 21.Le récipient 14 a une structure symétrique par rapport au plan DD' vertical passant par l'axe de la cuve 2. Le fond 16 comporte deux bords opposés 22a et 22b, parallèles , la forme du fond 16 entre lesdits bords 22a, 22b pouvant être semi-circulaires, s'adaptant à la forme intérieure cylindrique de la partie basse de la cuve 2. Les deux trappes de déchargement 17a et 17b sont montées pivotantes par l'intermédiaire d'axes de rotation 23a, 23b fixés selon les bords 22a et 22b.

Le jeu de tringles 18,19,2O est articulé autour de deux axes de rotation 24,25. Un premier jeu de tringles 18a, 18b est fixé entre le premier axe de rotation 24 et chacune des trappes de déchargement 17a, 17b. Un deuxième jeu de tringles 19 relie le premier 24 et le second 25 axes de rotation. Un troisième jeu de tringles relie le second axe de rotation 25 et l'extrémité libre 26 de la tige 27 du vérin 21. L'axe de rotation 25 est fixe tandis que l'axe de rotation 24 est apte à se déplacer en hauteur. Les deuxième et troisième jeux de tringles 19,2O sont fixes en position l'un par rapport à l'autre, tandis que les articulations entre le premier et le deuxième jeu de tringles 18,19 ainsi que entre le troisième jeu de tringles 2O et la tige 27 du vérin 21 peuvent pivoter librement sur le plan angulaire. Le vérin 21 est lui-même apte à pivoter par rapport à son point d'ancrage 28.

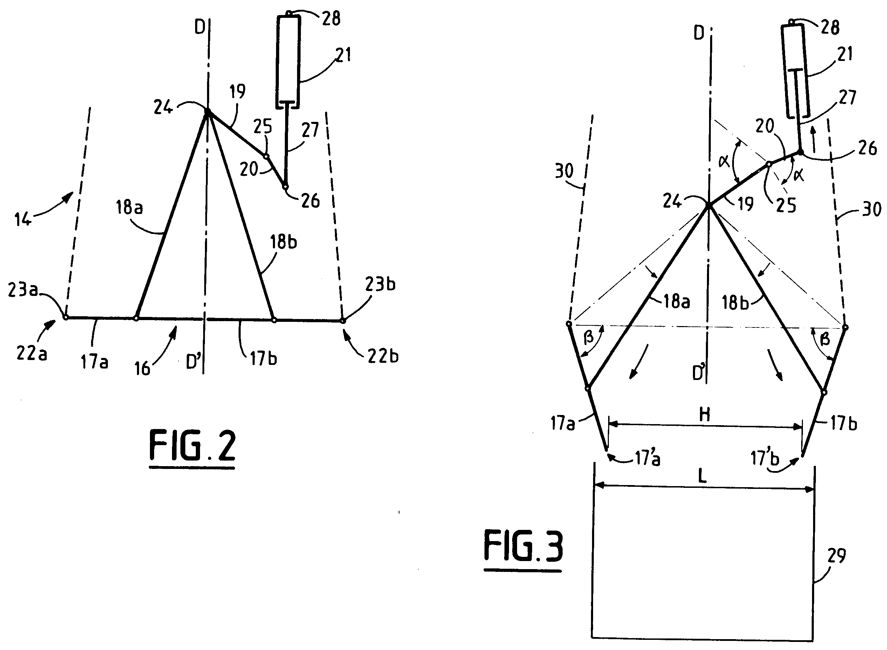

Le déchargement par gravité des déchets contenus dans le récipient perforé 14 dans le chariot 29 est réalisé par simple commande du vérin 21, après ouverture du couvercle de fond 6. On a représenté sur les figures 2 et 3 la disposition des différents organes correspondant pour la figure 2 au récipient 14 en position de stockage de déchets et pour la figure 3 au récipient 14 en position de déchargement.

En position de stockage de déchets, la tige 27 du vérin 21 étant sortie, le premier axe de rotation 24 est en position haute, de sorte que les deux trappes de déchargement 17a et 17b sont maintenues par le premier jeu de tringles 18a, 18b en position horizontale, dans le prolongement l'une de l'autre.

Pour passer à la position de déchargement, il suffit de commander la rentrée de la tige 27 du vérin 21. Ceci entraîne les pivotements d'angle α du troisième jeu de tringles 2O autour du second axe 25 et corrélativement le pivotement du même angle α du deuxième jeu de tringles 19 autour du même second axe 25. Au cours de ce déplacement, le premier axe 24, porté par le second jeu de tringles 19, passe en position basse . Cette descente provoque corrélativement la descente du premier jeu de tringles 18a, 18b et celle des deux trappes de déchargement 17a et 17b qui, étant maintenues par les axes de rotation 23a, 23b pivotent autour de ceux-ci d'un angle β.

La disposition relative et les dimensions des différents organes qui viennent d'être décrits sont déterminées en sorte que l'écartement H entre les deux bords libres 17'a et 17'b des deux trappes de déchargement 17a et 17b soient légèrement inférieur à la dimension L du chariot 29 qui est placé sous le récipient perforé 16 avant l'actionnement du vérin 21.

De préférence le troisième jeu de tringles 2O ainsi que le vérin 21 est extérieur à la cuve 2 tandis que les autres éléments sont disposés à l'intérieur de celle-ci, le second axe de rotation 25 traversant la paroi de la cuve de manière étanche.

Comme illustré aux figures 2 et 3, la paroi latérale perforée 3O du récipient 14 est légèrement inclinée par rapport au fond 16, de manière divergente depuis la partie haute dudit récipient 14. L'angle d'inclinaison est de quelques degrés. Cette disposition particulière a pour but de permettre un meilleur décrochement par gravité des déchets broyés en contact avec ladite paroi latérale 3O lors de l'ouverture des deux trappes de déchargement 17a, 17b.

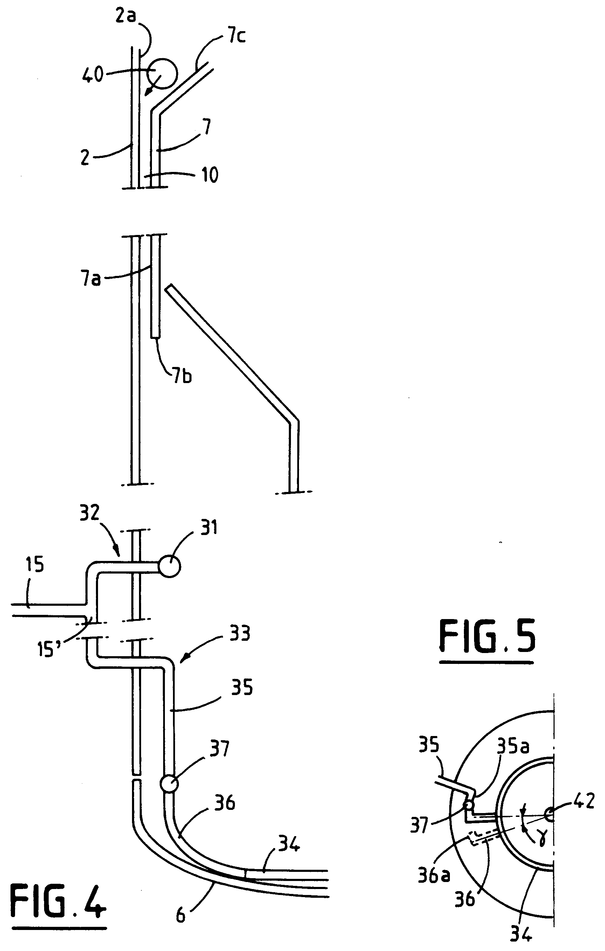

Comme indiqué ci-dessus, la conduite 15 d'alimentation en vapeur se prolonge à l'intérieur de la cuve 2 sous la forme d'une conduite 31 qui est circulaire, faisant le tour de la cuve au niveau de la paroi latérale 3O perforés du récipient 14. La même conduite 15 se prolonge également dans le couvercle de fond 6. On a représenté à la figure 4 ces deux circuits internes d'alimentation en vapeur, à savoir le premier circuit 32 d'injection dans le corps de la cuve 2 et le second circuit 33 correspondant à l'injection dans le couvercle de fond 6. S'agissant notamment du second circuit 33, il comporte une conduite circulaire 34 qui est fixée sur le fond du couvercle 6 et qui est raccordée à la conduite d'alimentation 15 par deux tronçons indépendants 35,36. Le premier tronçon 35 relie la conduite d'alimentation 15 depuis une jonction 15', passe à travers la paroi de la cuve 2 de manière étanche et descend vers le couvercle de fond 6 pour se terminer par un embout 37 de raccordement. Il est à noter que l'extrémité de la conduite 35, avant l'embout 37, présente un coude de sorte que ladite extrémité 35a est sensiblement horizontale. Le second tronçon 36 raccorde la conduite circulaire 34 fixée sur le couvercle de fond 6. Elle remonte vers la périphérie dudit couvercle et présente à son extrémité un coude de sorte que ladite extrémité 36a est sensiblement horizontale. L'embout de raccordement 37, les dimensions relatives des deux tronçons 35,36 à leurs extrémités 35a et 36a sont telles que l'extrémité 36a peut pénétrer dans l'embout 37 de raccordement et relier de manière parfaitement étanche les deux tronçons 35,36 lors de la fermeture et du verrouillage du couvercle de fond 6.

Dans l'exemple illustré, le couvercle de fond 6 est à basculement vertical, c'est-à-dire qu'il est monté pivotant par rapport à un axe fixe 38 grâce à un vérin 39. Afin d'obtenir une parfaite étanchéité entre le couvercle de fond et la cuve , malgré la pression de la vapeur, le verrouillage du couvercle 6 est réalisé par des moyens de verrouillage connus qui s'enclenchent grâce à la rotation sur lui-même dudit couvercle 6 d'un angle γ déterminé. Cette rotation est notamment obtenue à l'aide de vérins.

C'est cette rotation d'angle γ qui est mise à profit pour raccorder de manière étanche le second circuit 33 d'alimentation en vapeur du couvercle de fond 6 au circuit d'alimentation 15. En effet lors de la fermeture du couvercle de fond 6 grâce à l'action du vérin 39, le tronçon 36 a la position qui est montrée en pointillés sur la figure 5, c'est-à-dire que son extrémité 36a se trouve à la même hauteur que l'extrémité 35a du premier tronçon 35 mais éloignée de celle-ci ; grâce à la rotation sur lui-même du couvercle 6 d'un angle γ, on obtient le rapprochement des deux extrémités 35a et 36a jusqu'à pénétration de l'extrémité 36a dans l'embout de raccordement 37.

Les deux conduites circulaires 31, 34 sont percées d'orifices permettant l'injection de la vapeur à l'intérieur de la cuve 2.

L'installation 1 comporte un circuit de refroidissement par eau. Ce circuit est composé d'une rampe de distribution d'eau, rampe disposée dans la partie haute de la cuve 2 et alimentée en eau sous pression grâce à une conduite 41. La rampe 4O est une rampe circulaire qui est logée près de la paroi interne 2a de la cuve 2 et de la paroi externe 7a de l'enceinte supérieure 7. On a représenté à la figure 4 la disposition prise par la rampe 4O. Les orifices de passage de l'eau sont dirigés vers la paroi interne 2a de la cuve 2 de manière à créer un ruissellement dans l'espace annulaire 1O entre la cuve 2 et l'enceinte supérieure 7, ruissellement qui préférentiellement se poursuivra le long de ladite paroi 2a au-delà de la partie basse 7b de l'enceinte inférieure 7 jusqu'au couvercle de fond 6.

Etant donné que l'écartement entre les deux parois 2a, 7a est faible, de l'ordre du centimètre, de préférence l'extrémité haute 7c de l'enceinte supérieure présente un décrochement vers l'intérieur de la cuve 2, comme montré sur la figure 4, de manière à permettre le placement de la rampe 4O de distribution d'eau. Le couvercle de fond 6 est muni d'une ouverture 42 qui est reliée à un circuit de vidange 43.

L'installation 1 comporte également un circuit 44 d'évacuation de la vapeur. Ce circuit 44 comporte deux branches l'une 44a correspond à l'évacuation de la vapeur lors de la mise en pression et l'autre 44b correspond à l'évacuation de la vapeur en fin de cycle. Autant la branche 44b du circuit 44 peut être évacuée librement, autant la branche 44a doit préalablement être soumise à décontamination. Pour cela on interpose sur la branche 44a un filtre 45 apte à décontaminer la vapeur qui s'échappe de la cuve après avoir été mis en contact avec les déchets alors que ceux-ci n'ont pas encore subi totalement le traitement de décontamination. Ce filtre comporte un média filtrant adapté à cet usage, qui peut être régénéré par une action complémentaire de la vapeur à contre-courant. Pour ce faire la branche 44a du circuit d'évacuation 44 comporte une alimentation 46 de vapeur en sorte que ladite vapeur peut traverser le média-filtrant à contre-courant. Le filtre est équipé d'un réservoir de stockage temporaire des condensats formés lors de la régénération du filtre. Ce réservoir 47 est raccordé jusqu'à la cuve 2.

L'installation 1 peut également comporter un circuit de mise partielle sous vide 48.

Le mode opératoire utilisé pour le broyage et la décontamination d'un lot déterminé de déchets est le suivant.

Le couvercle de chargement 5 étant ouvert on introduit par l'ouverture supérieure 3 les déchets dans la cuve 2, ceux-ci s'accumulant dans l'enceinte supérieure 7. Ces déchets peuvent être de tous ordres, étant notamment contenus dans des sacs plastiques. Il peut s'agir de seringues, de petits matériels médicaux ou chirurgicaux, de matériaux absorbants du type hygiénique, de couche-culottes... Une fois ces déchets introduits dans l'enceinte supérieure 7, le couvercle de chargement 5 est refermé de manière étanche.

L'ensemble des opérations qui vont être maintenant décrites sont de préférence réalisées automatiquement grâce à un automate programmable qui est connecté à l'ensemble des organes de l'installation.

Après fermeture du couvercle de chargement 5, le broyeur 8 est mis en marche. L'alimentation du broyeur 8 se fait simplement par gravité, les portions relevées 11 permettant de collecter vers le broyeur 8 l'ensemble des déchets accumulés dans l'enceinte supérieure 7. Il peut être prévu un système de dévoutage 13 déplaçant les déchets , notamment lorsqu'il s'agit de sacs plastiques afin d'éviter une accumulation localisée desdits sacs et d'obtenir une alimentation régulière du broyeur.

Simultanément au broyage, la mise en chauffe de la cuve 3 est commencée par introduction de vapeur jusqu'à un premier palier de température , par exemple de l'ordre de 50°C.

Les déchets broyés sont pendant toute l'opération de broyage tombés directement dans le récipient perforé 14 où ils se sont accumulés. A la fin du broyage on injecte de la vapeur pour atteindre au coeur de la matière un second palier de température, notamment de l'ordre de 138°C. Une fois que ce seuil est atteint, il est maintenu pendant un temps de 1Omn. C'est ce seuil de température et cette durée qui permettent de décontaminer les déchets broyés.

Lors de la montée en température, on constate bien sûr une augmentation de pression. On régule la pression jusqu'à un seuil déterminé, notamment de l'ordre de 3,8 bars. Cette régulation est obtenue en évacuant en partie la vapeur par la branche 44a du circuit d'évacuation 44. C'est cette vapeur qui passe sur le média-filtant contenu dans le filtre 45.

Le contrôle de la température peut être effectué grâce à une sonde non représentée qui pénètre au coeur des déchets broyés contenus dans le récipient perforé 14. Après la fin de la période de décontamination, on procède au refroidissement de la partie basse de la cuve 2. Ce refroidissement est obtenu par injection dans la rampe de distribution 4O d'eau sous pression. Comme indiqué précédemment, cette eau est injectée dans l'espace annulaire 1O prévu entre la cuve 2 et l'enceinte supérieure 7, elle ruisselle dans cet espace annulaire puis le long de la paroi interne 2a de la cuve 2 jusque dans le couvercle de fond 6. On comprend que l'eau doit être sous pression compte-tenu de la pression de vapeur régnant à l'intérieur de la cuve 2. Grâce à ce ruissellement , on obtient un refroidissement jusqu'à un troisième palier de température qui est par exemple de l'ordre de 6O°C.

Ce palier doit être à un niveau suffisamment bas pour permettre l'ouverture de la cuve sans préjudicie pour l'opérateur.

Une fois ce palier atteint, les condensats de vapeur, les rejets liquides provenant des déchets et l'eau de refroidissement qui se sont accumulés dans le couvercle de fond 6 sont vidangés par le circuit 43 vers une cuve de rejet.

On réalise un vide partiel dans la cuve grâce au circuit 48 de manière à condenser la vapeur résiduelle.

On ouvre le couvercle de fond 6 en actionnant le vérin 39.

L'opérateur présente le chariot 29 sous l'ouverture de déchargement 4.

Le vérin 21 est actionné en sorte de rentrer sa tige 27 et de commander l'abaissement des deux trappes de déchargement 17a et 17b, ce qui provoque la chute par gravité des déchets broyés et décontaminés qui se trouvent dans le récipient perforé 14. Une fois le récipient vidé, l'opérateur déplace le chariot 29, le couvercle de fond 6 est refermé. Eventuellement on procède à un nouveau refroidissement de l'enceinte supérieure 7 par distribution d'eau à partir de la rampe 4O, l'eau ruisselant à l'intérieur de la cuve 2 étant évacuée par le circuit vidange 43. Ce refroidissement complémentaire a pour but d'abaisser la température intérieure de l'enceinte supérieure 7 pour permettre le chargement du lot suivant de déchets sans préjudice pour l'opérateur.

Il est procédé à la régénération du média-filtrant contenu dans le filtre 45 par injection de vapeur à contre-courant dans ce filtre. Les condensats résultants sont accumulés dans le récipient de stockage 47. Ils ne sont injectés dans la cuve 2 qu'après la fin du cycle de traitement d'un lot déterminé. En d'autres termes ils sont introduits dans la cuve 2 et séjournent dans la couvercle de fond 6 en début du cycle de traitement du lot suivant ; ils seront décontaminés avec les liquides éventuels s'écoulant des déchets broyés lors du traitement thermique de décontamination de ce lot suivant. Ainsi grâce à ce mode opératoire, plus aucun élément contaminé se sort de l'installation.

La présente invention n'est pas limitée au mode de réalisation qui vient d'être décrit à titre d'exemple non exhaustif. En particulier l'ouverture du couvercle de fond peut se faire , non plus par un basculement vertical, mais par une rotation dans un plan sensiblement horizontal ; dans ce cas, la connexion par emmanchement se fait, non plus à l'intérieur, mais à l'extérieur de la cuve. Le choix entre l'une ou l'autre de ces deux solutions est à prendre en fonction de l'espace libre laissé autour de l'installation. De plus, dans le cas d'un couvercle à basculement vertical, le verrouillage peut se faire, non plus par rotation sur lui-même d'un angle déterminé, mais par rotation d'une couronne extérieure de verrouillage ; on a alors un emmanchement vertical des tronçons de raccordement à la conduite d'alimentation. D'autre part dans l'eau de circuit de refroidissement , il est possible d'injecter un désodorisant destiné à masquer les odeurs provenant des déchets.

Enfin le circuit de refroidissement par eau de l'installation peut différer de celui décrit ci-dessus. Par exemple la structure de la cuve peut être à double enveloppe et la rampe de distribution peut être disposée dans la partie haute de la cuve en sorte de créer un ruissellement dans l'espace entre les deux enveloppes. L'eau de refroidissement est collectée à la partie basse, extérieure, de la cuve ; elle n'est jamais en contact avec le milieu contaminé.

## Revendications

1. Installation comportant une enceinte supérieure d'alimentation des déchets (7) , une enceinte inférieure (9) de réception, de traitement et d'évacuation des déchets broyés, des moyens de broyage (8) intercalés entre les deux enceintes, des moyens d'injection de vapeur dans l'enceinte inférieure, et des moyens de commande de l'ouverture et de la fermeture d'une trappe de déchargement du fond de l'enceinte inférieure, caractérisée en ce qu'elle comporte également un récipient (14) de stockage des déchets broyés qui est perforé pour permettre le passage de l'eau et de la vapeur , qui est ouvert sur le dessus et qui est disposé dans l'enceinte inférieure (9) sous les moyens de broyage (8), en ce que le fond (16) dudit récipient (14) de stockage des déchets comporte au moins une trappe de déchargement (17a, 17b), en ce que l'enceinte inférieure (9) comporte un couvercle de fond (6) et en ce que ladite installation comporte des moyens de commande de l'ouverture et de la fermeture de la ou des trappes de déchargement (17a, 17b) ainsi que des moyens de commande de l'ouverture et de la fermeture du couvercle de fond (6) de l'enceinte inférieure (9).

2. Installation selon la revendication 1 caractérisée en ce que le fond (16) du récipient de stockage (14) comporte deux trappes symétriques (17a, 17b) de déchargement , chacune étant pivotante par rapport à un axe de pivotement (23a, 23b) qui est monté selon l'un des bords (22a, 22b) du fond (16) du récipient (14) , et en ce que les deux trappes de déchargement (17a, 17b) sont actionnées par un jeu de tringles (18,19,2O) commandé par un vérin (27) disposé à l'extérieur de l'enceinte inférieure (9).

3. Installation selon l'une des revendications 1 ou 2 caractérisée en ce que les montants latéraux (3O) du récipient de stockage (14) sont inclinés de manière divergente depuis l'ouverture supérieure vers le fond (16) du récipient (14).

4. Installation selon la revendication 1 caractérisée en ce que les moyens d'injection de la vapeur dans l'enceinte inférieure (9) comprennent un premier circuit (31,32) autour du récipient de stockage (14) des déchets et un second circuit (33,34) qui est disposé dans le couvercle de fond (6).

5. Installation selon la revendication 4 caractérisée en ce que les premiers et second circuits d'injection de vapeur sont connectés au même circuit d'alimentation en vapeur (15) et sont raccordés l'un à l'autre lorsque le couvercle de fond (6) est en position fermée.

6. Installation selon la revendication 5 caractérisée en ce que le couvercle de fond (6) étant à fermeture par basculement selon un axe horizontal (38) et à verrouillage par rotation sur lui-même d'un angle déterminé (γ) ; le circuit d'alimentation (35) pénétrant à l'intérieur de l'enceinte inférieure (9) se termine par un premier embout (37) de raccordement et le second circuit commence par un second embout de raccordement, les deux embouts étant conformés et disposés de telle sorte que après fermeture et rotation de verrouillage du couvercle l'un des embouts est emmanché dans l'autre de manière étanche.

7. Installation selon la revendication 1 caractérisée en ce qu'elle comporte une cuve (2) à l'intérieur de laquelle sont disposés, dans sa partie haute, l'enceinte supérieure (7) et dans sa partie intermédiaire les moyens de broyage (8) ; l'enceinte inférieure (9) étant constituée au moins en partie par la partie basse de la cuve (2).

8. Installation selon l'une des revendications 1 ou 7 caractérisée en ce qu'elle comporte des moyens de refroidissement qui consistent en une rampe (4O) de distribution d'eau sous pression qui est disposée dans la partie haute de l'installation et qui distribue l'eau selon la face externe (7a) de l'enceinte supérieure (7) en sorte que l'eau ruisselle jusque dans le couvercle de fond (6) de l'enceinte inférieure (9) sans pénétrer dans les moyens de broyage (8).

9. Installation selon la revendication 8 caractérisée en ce que l'enceinte supérieure (7) et la cuve (2) étant de forme cylindrique et coaxiales, la rampe de distribution d'eau (4O) est une rampe circulaire qui distribue l'eau dans l'espace annulaire (1O) compris entre l'enceinte supérieure (7) et la cuve (2) , et en ce que les moyens de broyage (8) sont d'une dimension inférieure au diamètre de la cuve (2).

10. Installation selon l'une des revendications 1 ou 7 caractérisée en ce que la cuve est à double enveloppe et en ce qu'elle comporte des moyens de refroidissement qui consistent en une rampe de distribution d'eau qui est disposée dans la partie haute de la cuve et qui distribue l'eau entre les deux enveloppes en sorte que l'eau ruisselle entre les deux enveloppes jusque dans la partie basse de la cuve, où elle est collectée, sans pénétrer dans la cuve.

11. Installation selon la revendication 1 caractérisée en ce qu'elle comporte :
a) un circuit (44a) d'évacuation de la vapeur contaminée équipé d'un filtre du type bactériologique et
b) un circuit de régénération du filtre comprenant une alimentation (46) de vapeur à contre-courant et un récipient de stockage (47) des condensats de régénération , ledit récipient de stockage (47) débouchant dans l'enceinte inférieure (9) et
c) un circuit de commande qui est programmé en sorte d'injecter le contenu du récipient de stockage des condensats dans l'enceinte inférieure (9) avant l'injection de vapeur à haute température.

## Claims

1. An installation comprising an upper enclosure (7) for feeding waste, a lower enclosure (9) for receiving, treating, and evacuating fragmented waste, fragmenter means (8) interposed between the two enclosures, means for injecting steam into the lower enclosure, and control means for opening and closing a discharge hatch at the bottom of the lower enclosure, the installation being characterized in that it also comprises a receptacle (14) for storing fragmented waste, which receptacle is perforated to pass water and steam, is open on top, and is disposed inside the lower enclosure (9) beneath the fragmenter means (8), in that the bottom of said waste storage receptacle (14) includes at least one discharge hatch (17a, 17b), in that the lower enclosure (9) includes a bottom cover (6), and in that said installation includes control means for opening and closing the discharge hatch(es) (17a, 17b) and control means for opening and closing the bottom cover (6) of the lower enclosure (9).

2. An installation according to claim 1, characterized in that the bottom (16) of the storage receptacle (14) has two symmetrical discharge hatches (17a, 17b), each pivoting about a respective pivot axis (23a, 23b) which is mounted on a respective edge (22a, 22b) of the bottom (16) of the receptacle (14), and in that the two discharge hatches (17a, 17b) are actuated by a set of rods (18, 19, 20) controlled by an actuator (27) disposed outside the lower enclosure (9).

3. An installation according to claim 1 or 2, characterized in that the lateral uprights (30) of the storage receptacle (14) are inclined so as to diverge from the top opening towards the bottom (16) of the receptacle (14).

4. An installation according to claim 1, characterized in that the means for injecting steam into the lower enclosure (9) comprise a first circuit (31, 32) around the waste storage receptacle (14) and a second circuit (33, 34) disposed in the bottom cover (6).

5. An installation according to claim 4, characterized in that the first and second steam injection circuits are connected to a common steam supply circuit (15) and are connected to each other when the bottom cover (6) is in the closed position.

6. An installation according to claim 5, characterized in that the bottom cover (6) closes by tilting about a horizontal axis (38) and locks by rotating about its own axis through a determined angle (γ); the feed circuit (35) which penetrates to the inside of the lower enclosure (9) terminating via a first coupling endpiece (37), and the second circuit beginning by a second coupling endpiece, the two endpieces being shaped and located in such a manner that after the cover has been closed and rotated into its locked position, one of the endpieces is sealingly engaged in the other.

7. An installation according to claim 1, characterized in that it includes a vessel (2) within which there are disposed: in the upper portion thereof, the upper enclosure (7); and in the bottom portion thereof, the fragmenter means (8); the lower enclosure (9) being constituted at least in part by the bottom portion of the vessel (2).

8. An installation according to claim 1 or 7, characterized in that it includes cooling means consisting in a distributor strip (40) for distributing water under pressure and disposed inside the upper portion of the installation, said strip distributing water on the outside face (7a) of the upper vessel (7) so that the water trickles down into the bottom cover (6) of the lower vessel (9) without penetrating into the fragmenter means (8).

9. An installation according to claim 8, characterized in that the upper enclosure (7) and the vessel (2) are cylindrical in shape and coaxial, and the water distribution strip (40) is circular in shape and distributes water into the annular gap (10) between the upper enclosure (7) and the vessel (2), and in that the fragmenter means (8) are smaller than the diameter of the vessel (2).

10. An installation according to claim 1 or 7, characterized in that the vessel is double-walled and in that it includes cooling means which comprise a water distribution strip disposed in the top portion of the vessel and which distributes water between the two walls so that the water tickles between the two walls down to the bottom portion of the vessel, where it is collected without penetrating into the vessel.

11. An installation according to claim 1, characterized in that it includes:
a) a circuit (44a) for evacuating contaminated steam, which circuit is fitted with a bacteriological type filter;
b) a filter regenerator circuit comprising a counterflow steam feed (46) and a receptacle (47) for storing the condensates from regeneration, said storage receptacle (47) opening out into the lower vessel (9); and
c) a control circuit which is programmed to inject the contents of the condensate storage receptacle into the lower vessel (9) before injecting high temperature steam.

## Patentansprüche

1. Anlage mit einem oberen Behälter (7) zur Zufuhr von Abfällen, einem unteren Behälter (9) zur Aufnahme, Behandlung und Ableitung von zermahlten Abfällen, einer Mahleinrichtung (8), die zwischen den beiden Behältern angeordnet ist, einer Einrichtung zum Dampfeinblasen in den unteren Behälter und einer Einrichtung zur Steuerung des Öffnens und Schließens einer Auslaßklappe des Bodens des unteren Behälters, dadurch gekennzeichnet, daß sie weiter einen Aufnahmebehälter (14) für die zermahlenen Abfälle umfaßt, der perforiert ist, um den Durchgang von Wasser und Dampf zu gestatten, und oben offen und im unteren Behälter (9) unter der Mahleinrichtung (8) angeordnet ist, daß der Boden (16) des Aufnahmebehälters (14) für die Abfälle mindestens eine Auslaßklappe (17a, 17b) umfaßt, daß der untere Behälter (9) eine Bodenkappe (6) aufweist und daß die Anlage eine Einrichtung zur Steuerung des Öffnens und Schließens der Auslaßklappe oder der Auslaßklappen (17a, 17b) sowie eine Einrichtung zur Steuerung des Öffnens und Schließens der Bodenkappe (6) des unteren Behälters (9) umfaßt.

2. Anlage nach Anspruch 1, dadurch gekennzeichnet, daß der Boden (16) des Aufnahmebehälters (14) zwei symmetrische Auslaßklappen (17a, 17b) umfaßt, die jeweils bezüglich einer entlang einer der Kanten (22a, 22b) des Bodens (16) des Behälters (14) befestigten Schwenkachse (23a, 23b) verschwenkbar sind, und daß die beiden Auslaßklappen (17a, 17b) durch einen Satz Stangen (18, 19, 20) betätigt werden, die durch einen außerhalb des unteren Behälters (9) angeordneten Zylinder (27) gesteuert werden.

3. Anlage nach einem der Ansprüche 1 oder 2, dadurch gekennzeichnet, daß die beiden Seitenwände (30) des Aufnahmebehälters (14) von der Öffnung oben zum Boden (16) des Behälters hin divergent geneigt sind.

4. Anlage nach Anspruch 1, dadurch gekennzeichnet, daß die Einrichtung zum Dampfeinblasen in den unteren Behälter (9) eine erste Leitung (31, 32) um den Aufnahmebehälter (14) für die Abfälle herum und eine zweite Leitung (33, 34) umfaßt, die in der Bodenkappe (6) angeordnet ist.

5. Anlage nach Anspruch 4, dadurch gekennzeichnet, daß die erste und zweite Dampfeinblasleitung an dieselbe Dampfversorgungsleitung (15) angeschlossen und miteinander verbunden sind, wenn sich die Bodenkappe (6) in geschlossener Stellung befindet.

6. Anlage nach Anspruch 5, dadurch gekennzeichnet, daß die Bodenkappe (6) durch Kippen entlang einer horizontalen Achse (38) geschlossen und durch Rotation um einen bestimmten Winkel (γ) um sich selbst verriegelt wird und die Versorgungsleitung (35), die in das Innere des unteren Behälters (9) hinein verläuft, mit einem ersten Anschlußstück (37) abschließt und die zweite Leitung mit einem zweiten Anschlußstück beginnt, wobei die beiden Anschlußstücke einander angepaßt und derart angeordnet sind, daß eines der Anschlußstücke nach Schließen und Verriegelung durch Rotation der Kappe in dichter Weise auf das andere aufgesteckt ist.

7. Anlage nach Anspruch 1, dadurch gekennzeichnet, daß sie ein Gefäß (2) umfaßt, in dessen Innerem im oberen Abschnitt der obere Behälter (7) und im mittleren Abschnitt die Mahleinrichtung (8) angeordnet sind, wobei der untere Behälter (9) zumindest teilweise durch den unteren Abschnitt des Gefäßes (2) gebildet wird.

8. Anlage nach einem der Ansprüche 1 oder 7, dadurch gekennzeichnet, daß sie eine Kühleinrichtung umfaßt, die aus einem Verteiler (40) von Druckwasser besteht, der im oberen Abschnitt der Anlage angeordnet ist und das Wasser entlang der Außenseite (7a) des oberen Behälters (7) verteilt, so daß es bis in die Bodenkappe (6) des unteren Behälters (9) läuft, ohne in die Mahleinrichtung (8) zu dringen.

9. Anlage nach Anspruch 8, dadurch gekennzeichnet, daß der obere Behälter (7) und das Gefäß (2) zylindrisch geformt und koaxial sind und der Verteiler (40) des Wassers ein kreisförmiger Verteiler ist, der das Wasser im ringförmigen Raum (10) zwischen dem oberen Behälter (7) und dem Gefäß (2) verteilt, und daß die Mahleinrichtung (8) von geringerer Abmessung ist als der Durchmesser des Gefäßes (2).

10. Anlage nach einem der Ansprüche 1 oder 7, dadurch gekennzeichnet, daß das Gefäß zweiwandig ist und eine Kühleinrichtung umfaßt, die aus einem Verteiler von Wasser besteht, der im oberen Abschnitt des Gefäßes angeordnet ist und das Wasser zwischen den beiden Wänden derart verteilt, daß es, ohne in das Gefäß zu dringen, zwischen den beiden Wänden bis in den unteren Abschnitt des Gefäßes läuft, wo es aufgefangen wird.

11. Anlage nach Anspruch 1, dadurch gekennzeichnet, daß sie folgendes aufweist:
a) eine Leitung (44a) zur Ableitung des kontaminierten Dampfes, die mit einem bakteriologischen Filter ausgestattet ist,
b) eine Leitung zur Regeneration des Filters mit einer Dampfversorgung (46) im Gegenstrom und einem Aufnahmebehälter (47) für das Regenerations-Kondenswasser, wobei der Aufnahmebehälter (47) in den unteren Behälter (9) mündet, und
c) eine Steuerschaltung, die so programmiert ist, daß der Inhalt des Aufnahmebehälters für das Kondenswasser vor dem Einblasen des heißen Dampfes in den unteren Behälter (9) gespritzt wird.
